(19) Europäisches Patentamt / European Patent Office / Office européen des brevets

(11) **EP 4 281 767 B1**

(12) **EUROPEAN PATENT SPECIFICATION**

(45) Date of publication and mention of the grant of the patent: **30.09.2026 Bulletin 2026/40**

(21) Application number: **22713276.8**

(22) Date of filing: **21.01.2022**

(51) International Patent Classification (IPC): ***G01N 33/487*** *(2006.01)* ***G01N 27/22*** *(2006.01)*

(52) Cooperative Patent Classification (CPC): **G01N 33/48735; G01N 27/221**

(86) International application number: **PCT/EP2022/051300**

(87) International publication number: **WO 2022/157282 (28.07.2022 Gazette 2022/30)**

(54) **APPLICATION OF PERMITTIVITY MEASUREMENT PROBES IN CELL SUSPENSIONS COMPRISING CELL AGGREGATES**

ANWENDUNG VON PERMITTIVITÄTSMESSSONDEN IN ZELLSUSPENSIONEN MIT ZELLAGGREGATEN

APPLICATION DE SONDES DE MESURE DE LA PERMITTIVITÉ DANS DES SUSPENSIONS CELLULAIRES COMPRENANT DES AGRÉGATS CELLULAIRES

(84) Designated Contracting States: **AL AT BE BG CH CY CZ DE DK EE ES FI FR GB GR HR HU IE IS IT LI LT LU LV MC MK MT NL NO PL PT RO RS SE SI SK SM TR**

(30) Priority: **21.01.2021 EP 21152718**

(43) Date of publication of application: **29.11.2023 Bulletin 2023/48**

(73) Proprietors:
- **Sartorius Stedim Biotech GmbH 37079 Göttingen (DE)**
- **Repairon GmbH 37079 Göttingen (DE)**

(72) Inventors:
- **HAUPT, Luis 6216 RP, Maastricht (NL)**
- **HUPFELD, Julia 37139 Adelebsen (DE)**

(74) Representative: **Schiweck Weinzierl Koch Patentanwälte Partnerschaft mbB Ganghoferstraße 68 B 80339 München (DE)**

(56) References cited:
**WO-A1-2022/065401** **US-A1- 2015 019 140**

- **LEAH M. ALLEN: "Serum-Free Culture of Human Mesenchymal Stem Cell Aggregates in Suspension Bioreactors for Tissue Engineering Applications", STEM CELLS INTERNATIONAL, vol. 2019, 7 November 2019 (2019-11-07), pages 1 - 18, XP093156151, ISSN: 1687-966X, Retrieved from the Internet <URL:http://downloads. hindawi.com/journals/sci/2019/4607461.xml> DOI: 10.1155/2019/4607461**
- **JUSTICE C ET AL: "Online- and offline-monitoring of stem cell expansion on microcarrier", CYTOTECHNOLOGY, vol. 63, no. 4, 12 May 2011 (2011-05-12), pages 325 - 335, XP019928801, ISSN: 1573-0778, DOI: 10.1007/ S10616-011-9359-4**
- **HAMILTON COMPANY INC: "Cell Density Applications eBook", 2018, XP055822649, Retrieved from the Internet <URL:https:// craft-sensors.s3.amazonaws.com/ Hamilton-Cell-Density-Applications-Ebook.pdf> [retrieved on 20210708]**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 4 281 767 B1

## Description

### CROSS-REFERENCE TO RELATED APPLICATIONS

The present application claims the benefit of priority of European Patent Application No. 21152718.9 filed 21 January 2021.

### TECHNICAL FIELD OF THE INVENTION

The present disclosure relates to a method of measuring cell density in a cell suspension comprising cell aggregates, the method comprising (i) Measuring the permittivity of the cell suspension; (ii) Comparing the measured permittivity with a predetermined value that is indicative of the cell density, thereby determining the cell density.

### BACKGROUND

It has been reported that the use of bioreactor systems enables production of large amounts of adherent cells such as PSCs, iPSCs and iPSC-derived cells (Kropp et al., 2017). In these systems, the cells usually do not attach to the surface of a dish but are grown in a free-floating suspension because adherent cells such as PSCs form aggregates when cultivated in suspension. Suspension culture in bioreactor systems is described to be more efficient than adherent culture because the culture can be monitored, controlled and automated even at high cell numbers and less material and amount of work is needed. Importantly, for these reasons the use of bioreactor systems would be preferred over static culture for GMP-controlled applications. Different bioreactor systems have been reported for suspension culture of adherent cells such as PSCs with stirred tank reactor (STR) systems being the best described ones. It was shown that high numbers of iPSCs and iPSC-CMs can be successfully generated in STR systems (Chen et al., 2012; Halloin et al., 2019; Hemmi et al., 2014; Jiang et al., 2019; Kempf et al., 2015; Kropp et al., 2016).

Additionally to monitoring the culture conditions in an adherent cell suspension culture, such as pH and dissolved oxygen (DO), the use of STRs also enables monitoring the quality of the cell culture itself. For this purpose, probes have been described for STRs that can be used for inline monitoring of the cells and for the control of the culture conditions. The dynamics of the cell concentration is an important parameter because it indicates the overall quality of the culture and may be used to control feeding and harvest. Without the use of inline probes, the cell concentrations can only be determined by regular sampling and offline measurements. So far, no inline measurement of cell density for cell suspension comprising cell aggregates has been described.

US2015/019140 discloses a permittivity probe measuring the permittivity of a cell suspension and comparing it with a predetermined value that is indicative of a biological property such as the cell density.

Accordingly, there is still a need for methods of measuring cell density in a cell suspension comprising cell aggregates. The present invention aims to address this need.

### SUMMARY OF THE INVENTION

This problem is solved by the subject-matter as defined in the claims. It is presented herein a method of measuring cell density in a cell suspension comprising cell aggregates, and a use of a permittivity probe in the method of the invention.

Accordingly, the present invention relates to a method of measuring cell density in a cell suspension comprising cell aggregates, the method comprising

(i) Measuring the permittivity of the cell suspension;
(ii) Comparing the measured permittivity with a predetermined value that is indicative of the cell density, thereby determining the cell density.

The present invention further relates to a use of a permittivity probe for determining the cell density of a suspension cell culture comprising cell aggregates.

The measuring (of step (i)) may be carried out in a bioreactor.

The bioreactor may be a stirred bioreactor, a rocking motion bioreactor and/or a multi parallel bioreactor.

The cell density of the suspension culture may be measured inline (in real time).

The measurement of the permittivity may be carried out using a permittivity probe. The permittivity measurement may be carried out by using dielectric spectroscopy.

The cells can be selected from the group consisting of, primary cells, cells obtained from a tissue or an organ, immortalized cells, stem cells such as pluripotent stem cells or cells derived from stem cells. The cells may be pluripotent

stem cells. The cells may also be pluripotent stem cells selected from the group consisting of induced pluripotent stem cells (iPSC), embryonic stem cells (ESC), parthenogenetic stem cells (pPSC) and nuclear transfer derived PSCs (ntPSC), preferably iPSCs.

The conversion factor may be obtained by

(a) Measuring the permittivity and the cell density at at least two, preferably at least three, different cell densities of a reference suspension culture;
(b) Correlating the measured cell permittivity of the reference suspension culture with the cell density, thereby determining the predetermined value that is indicative of the cell density.

The correlation may provide a linear correlation. The reference suspension culture and the suspension culture may be from a similar or the same or a similar cell type, cell line, tissue or organ. The reference suspension culture and the suspension culture may be cultured using the same culture medium. The reference suspension culture and the suspension culture may be cultured in a similar or the same bioreactor.

The cell density may be viable cell density.

The present invention further relates to a use of a permittivity probe in the method of the invention.

## BRIEF DESCRIPTION OF THE DRAWINGS

The invention will be better understood with reference to the detailed description when considered in conjunction with the non-limiting examples and the accompanying drawings, in which:

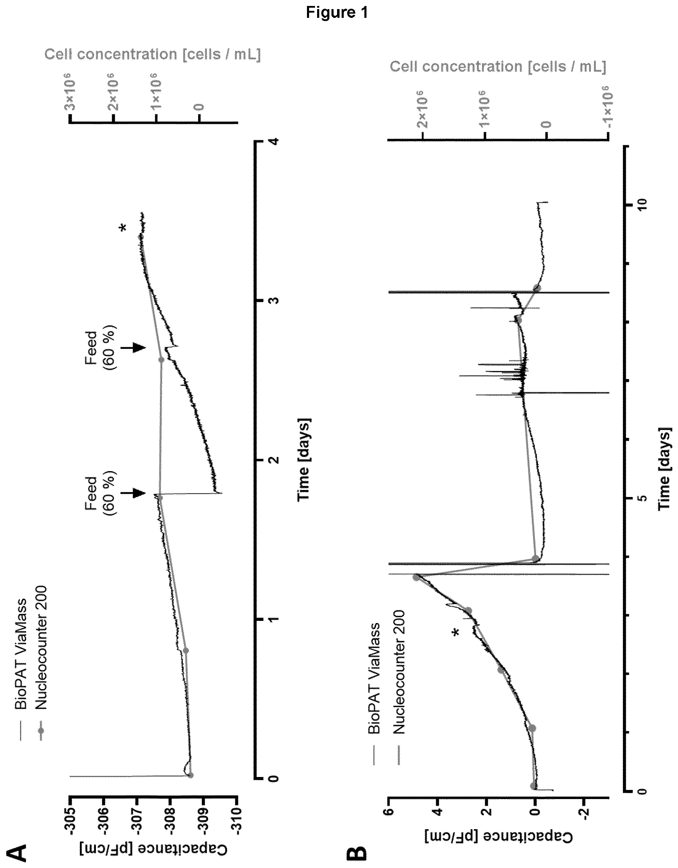

**Fig. 1** shows inline permittivity measurements (BioPAT® ViaMass) compared to offline cell counts (Nucleocounter 200). The permittivity measurement correlates with the cell concentration. Interestingly, the permittivity measurement recorded dynamic changes that were not captured by the offline measurements (Exemplary plateaus marked by asterisks and sudden medium addition marked by arrows.). **Fig. 1A** shows exemplary run No. 1: The culture volume was increased at the marked time points. **Fig. 1B** shows exemplary run No. 2: The culture volume was kept at a constant level by perfusion. The perfusion rate was increased on day 3 at the marked time when the capacitance measurement plateaued. On day 4 and 9, the iPSCs were passaged indicated by sudden strong changes in capacitance, which was caused by aspirating and refilling of the UniVessel.

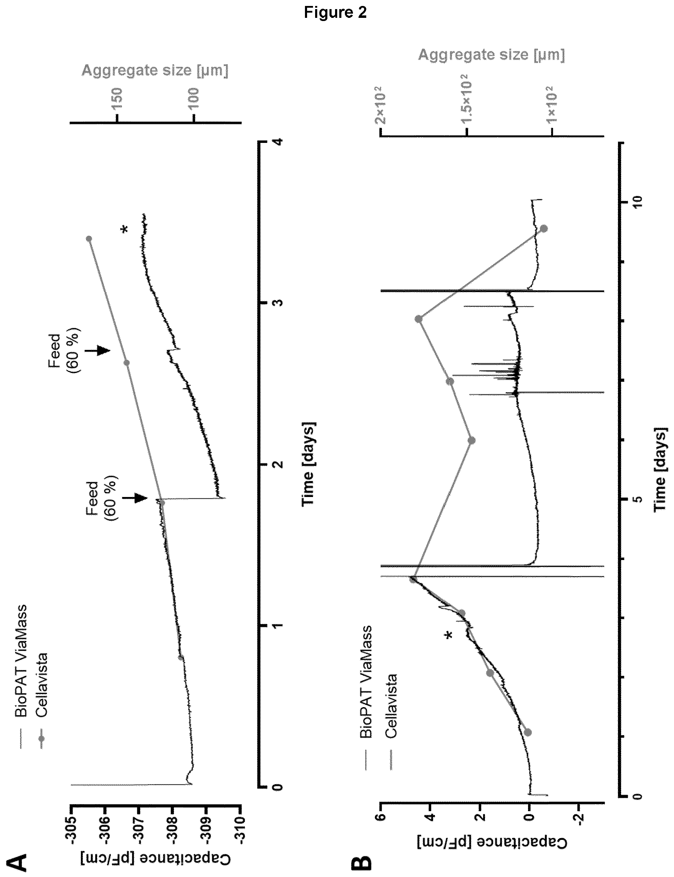

**Fig. 2** shows inline permittivity measurements (BioPAT® ViaMass) compared to offline analysis of aggregate size (Cellavista). The permittivity measurement does not correlate with the aggregate size. Alterations in culture volume are clearly represented in the capacitance recordings whereas the aggregate size is not affected by them. **Fig. 1A** shows exemplary run No. 1: The culture volume was increased at the marked time points. **Fig. 1B** shows exemplary run No. 2: The culture volume was kept at a constant level by perfusion. On day 4 and 9, the iPSCs were passaged indicated by the sudden strong changes in capacitance, which was caused by aspirating and refilling of the UniVessel.

## DETAILED DESCRIPTION OF THE INVENTION

The present invention is described in detail in the following and will also be further illustrated by the appended examples and figures.

So far, it was only shown that suspension culture of cells cultured on microcarriers allow for permittivity measurement and correlation to cell concentration. However, it is important to note that cell-only aggregate suspension culture and microcarrier-based suspension culture is not comparable. This is because cells grow as single or few layers on the microcarriers. On the other hand, cells in aggregates grow tightly in many layers and have a considerably higher amount of cell-cell interactions. The invention describes for the first time the successful application of a permittivity measurement probe in a cell-only, i.e. microcarrier-free, aggregate suspension culture of cells such as PSCs and the correlation to the cell concentration. As shown in Example 1, the measured permittivity correlates well with the cell density of PSC aggregates but surprisingly does not correlate with cell aggregate size. Thus, measuring permittivity can be used for determination of the cell density of cells in cell aggregates while it is not influenced by changes in aggregate size. The present invention enables the inline monitoring and assessment of PSC quality, proliferation and cell concentration in a suspension culture and will allow for the control of culture parameters and key process steps. Thereby, the invention will contribute to a GMP-controlled production of cells in large scales. Additionally, counting the number of cells in cell aggregates always includes the prior dissociation of the cell aggregates into single cells. When using the methods and uses of the invention, cell dissociation is no longer necessary for counting of the cells, which allows a direct monitoring of the cell density without any delay.

The general principle underlying the method of the present invention is the following: First, a predetermined value

that is indicative of the cell density or - in other words - which allows conversion of permittivity into cell density, has to be obtained. This can, e.g., be done by measuring the cell density obtaining a sample from the suspension culture, dissociating the cell aggregates and counting the cells using an "offline" method such as manual counting or using an automated cell counter. At the same time, the permittivity of the suspension culture is measured. This is repeated at least one more time at another cell density, e.g., by simply culturing the PSCs for a period of time to let the cell density increase. Based on these at least two data pairs, a correlation between permittivity and cell density can be obtained and thus the predetermined value can be obtained. This predetermined value can then be used to convert the inline permittivity measurements in inline cell density for any subsequent cultivation.

Accordingly, the present invention relates to a method of measuring cell density in a cell suspension comprising cell aggregates, the method comprising

(i) Measuring the permittivity of the cell suspension;
(ii) Comparing the measured permittivity with a predetermined value that is indicative of the cell density, thereby determining the cell density.

The "absolute permittivity", often simply called "permittivity" and denoted by the Greek letter $\varepsilon$ as used herein relates to a measure of the electric polarizability of a dielectric. A material with high permittivity polarizes more in response to an applied electric field than a material with low permittivity, thereby storing more energy in the material. The SI unit for permittivity is Farad per meter (F/m). In the context of the invention, the dielectric can be seen as the cell volume of viable cells. The cells, in particular the number of cells, have a measurable influence on cell permittivity, which can in principle be used to derive the cell number. However, a cell suspension is a rather complex electrical system and the person skilled in the art cannot expect that cell aggregates behave similarly to single cells. Nonetheless, the present inventors were successfully able to employ permittivity measurements for cell density determination also with cell aggregates.

As described herein (see also Example 1), the present inventors found that the cell density of the cell suspension can be measured inline or, in other words, in real time. Accordingly, the cell density of the suspension culture may be measured inline (in real time).

Permittivity of the cell suspension can be measured by various ways, which a person skilled in the art is aware of. One exemplary way is to use a permittivity probe. Accordingly, the measurement of the permittivity may be carried out using a permittivity probe. Permittivity probes are commercially available: e.g. BioPAT® ViaMass, available form Sartorius Stedim Biotech, Incyte, available from Hamilton, or Futura Probe, available from Aber Instruments Ltd.

In one embodiment, the permittivity measurement is carried out by using dielectric spectroscopy (DS). DS is based on the measurement of the passive dielectric properties of substances or biological units in a conducting medium. The term basically describes the measurement and analysis of the electrical capacitance and conductivity over a certain range of frequencies. The sample, called the dielectric, is placed in the electrical field between two electrodes. The change in the electric current-voltage relation in the presence of an alternating electrical field is then used to derive information on the sample. The basic idea of DS is to apply a periodically alternating electrical field, e.g., at various frequencies, to a system (a cell suspension comprising cell aggregates for example). The system for DS can be an entire multicellular organisms such as a human in medical applications of DS, or in the case of interest here a solution/suspension containing suspended or supported cells or unicellular organisms at least some of which are alive, along with low molecular solutes (salts, nutrients) and perhaps cell debris, virus, and virus particles. If the frequency is in the correct range, then some components in the medium can respond for example by storing some energy as temporarily separated charges (polarization). When the electrical field is periodically reversed then some lag in the system response is detected (amplitude and/or frequency changes). This response is the basis of dielectric spectroscopy.

The AC electrical field applied to the samples may cause, depending on the frequency and field strength, a polarization, orientation or displacement of electrically charged entities that may range from single inorganic ions to whole cells or even multi-cell organisms. In the range between 0.1 - 10 MHz, the method is termed radio frequency impedance spectroscopy (RFI) and the polarization of non conducting entities with surfaces, such as cell membranes, occurs. Accordingly, the frequency of the AC electrical field applied may be in the range between 50 kHz to 20 MHz, more preferably in a range of 300 to 900 kHz, more preferably in a range of 400 to 800 kHz, more preferably in a range of 500 to 700 kHz and most preferably at about 580 kHz. This range represents a small fraction of the wide range of frequencies possible with DS. Intermediate wavelengths cause the change of orientation of dipoles while near infra-red and infra-red frequencies cause atomic relaxation. Electronic relaxation is observed in the range of visible light. In the radio frequency range, cells with intact plasma membranes basically act as capacitors, since the non-conducting nature of the generally lipid-based cell plasma membrane allows the buildup of charge. Living organisms actively maintain electrochemical potential differences across their membranes. Further guidance on how to use dielectric spectroscopy can be found in Justice et al., 2011.

Capacitance values of viable cells with intact membrane are very high compared to nonviable cells, so that nonviable cells, leaking cells, cell debris, evolved gas bubbles and other media components are essentially invisible to RFI.

Accordingly, the cell density as used herein preferably is viable cell density (living cells/volume).

As described herein, the cell density is determined by comparing the measured permittivity with a predetermined conversion factor. This conversion factor can be obtained by correlating the measured permittivity of a reference suspension culture with the actual cell density of the reference suspension culture at different cell densities, e.g. obtained by manual or automated cell counting. Accordingly, the conversion factor described herein may be obtained by

(a) Measuring the permittivity and the cell density at at least two, preferably at least three, different cell densities of a reference suspension culture;

(b) Correlating the measured cell permittivity of the reference suspension culture with the cell density, thereby determining the predetermined value that is indicative of the cell density.

The correlation may be a linear correlation or, in other words, the correlation may provide a linear correlation. "Linear correlation" may in this context be understood as the result of a linear regression analysis. In statistics, linear regression is a linear approach to modeling the relationship between a scalar response (or dependent variable) and one or more explanatory variables (or independent variables). The case of one explanatory variable is called simple linear regression and applies to the linear regression described within this disclosure. In linear regression, the relationships are modeled using linear predictor functions whose unknown model parameters are estimated from the data. Such models are called linear models. Most commonly, the conditional mean of the response given the values of the explanatory variables (or predictors) is assumed to be an affine function of those values; less commonly, the conditional median or some other quantile is used. Like all forms of regression analysis, linear regression focuses on the conditional probability distribution of the response given the values of the predictors, rather than on the joint probability distribution of all of these variables, which is the domain of multivariate analysis. Linear regression models are often fitted using the least squares approach, but they may also be fitted in other ways, such as by minimizing the "lack of fit" in some other norm (as with least absolute deviations regression), or by minimizing a penalized version of the least squares cost function as in ridge regression ($L^2$-norm penalty) and lasso ($L^1$-norm penalty). Conversely, the least squares approach can be used to fit models that are not linear models. Thus, although the terms "least squares" and "linear model" are closely linked, they are not synonymous. However, the correlation is not limited to being linear but could also be a non-linear correlation or regression. In case a linear regression is used, a linear function like the equation shown in the following may be calculated:

$$f(x) = ax + b$$

wherein a is the slope of the line and b is the intercept. The function f(x) may give the cell density for each value of measured permittivity x. Thus, f(x) can be seen as the predetermined value.

The predetermined value is obtained by correlating at least two values, which is, e.g., the absolute minimum for a linear regression. However, the more data pairs (permittivity and cell density) are obtained, the exacter the result of the correlation is. Accordingly, preferably three, four, five, six, seven, eight, nine, ten or ten or more data pairs are obtained before performing correlation. Advantageously, the permittivity is obtained for a range of cell densities, which are to be expected during the complete cultivation process of the cell suspension.

Advantageously, the "reference suspension culture" is a cell suspension that is cultured under essentially the same conditions as the cell suspension to be assessed, e.g., by the methods or uses described herein. The same conditions may include, but are not limited to, the cell type, cell line, culture medium and/or the culture vessel used for cultivation such as a bioreactor. In some embodiments, the same conditions include the cell type, cell line, culture medium and the culture vessel used for cultivation such as a bioreactor.

Accordingly, the reference suspension culture and the suspension culture preferably are from a similar or the same cell type, cell line, tissue or organ. Similar in the context of cell types, cell lines, tissues or organs means that they are not necessarily obtained from the same subject but are the same cell type. PSCs obtained from two different patients can be seen as similar. On the other hand, cardiomyocytes and neurons from the same patient can be seen as not similar. PSCs obtained from the same patient can be seen as the same cells. A similar reasoning applies to cell lines. E.g., two PSC cell lines, whose origin lies in two different patients, can be seen as similar. A further preferred feature that describes a similar or the same cell type, cell line, tissue or organ is the differentiation state. Dielelectrical properties may change during differentiation stages. Accordingly, the cells of the cell suspension and the reference suspension culture preferably have the same differentiation stage. In case of PSCs, this may mean that the PSCs of the cell suspension and the reference suspension culture are not differentiated but remain in pluripotent state.

Additionally or alternatively, the reference suspension culture and the suspension culture are cultured using the same culture medium. The same culture medium is at least a culture medium having essentially the same concentration of salts and/or (preferably "and") essentially the same pH. Additionally or alternatively, the conductivity of the culture medium may be essentially the same. The same medium may also relate to (exactly) the same culture medium. It may also relate to

a medium that has essentially the same composition.

Furthermore additionally or alternatively, the reference suspension culture and the suspension culture preferably are cultured in a similar or the same bioreactor. A similar bioreactor means a bioreactor, which is the same model of a bioreactor or a bioreactor, which has essentially the same dimensions and whose culture vessel is made from the same material.

The term "suspension culture" or "cell suspension", both of which terms can be used interchangeably, as used herein is a type of cell culture in which single cells or small aggregates of cells are allowed to function and multiply in an preferably agitated growth medium, thus forming a suspension (c.f. the definition in chemistry: "small solid particles suspended in a liquid"). This is in contrast to adherent culture, in which the cells are attached to a cell culture container, which may be coated with proteins of the extracellular matrix (ECM). In suspension culture, in one embodiment no proteins of the ECM are added to the cells and/or the culture medium. The suspension culture preferably is essentially free of solid particles such as beads, microspheres, microcarrier particles and the like; cells or cell aggregates are no solid particles within this context. In one embodiment, the cells are not in microcarrier (suspension) culture. Preferably, the suspension culture is a perfusion suspension culture.

Adherent cells that are cultured in suspension, i.e. cannot attach to the culture vessel, may form cell aggregates. This also applies to the PSCs cultured in the uses and methods described herein. As used herein, the terms "aggregate" and "cell aggregate", which may be used interchangeably, refer to a plurality of cells such as (induced) pluripotent stem cells, in which an association between the cells is caused by cell-cell interaction (e.g., by biologic attachments to one another). Biological attachment may be, for example, through surface proteins, such integrins, immunoglobulins, cadherins, selectins, or other cell adhesion molecules. For example, cells may spontaneously associate in suspension and form cell-cell attachments (e.g., self-assembly), thereby forming aggregates. In some embodiments, a cell aggregate may be substantially homogeneous (i.e., mostly containing cells of the same type). In other embodiments, a cell aggregate may be heterogeneous, (i.e., containing cells of more than one type).

The methods and uses of the disclosure are suitable for cell aggregates. The cell aggregates may vary in size. The cell aggregates may have an average diameter between about 50 and 800 μm, between about 150 and 800 μm, of at least about 800 μm, of at least about 600 μm, of at least about 500 μm, of at least about 400 μm, of at least about 300 μm, of at least about 200 μm, of at least about 150 μm, between about 300 and 500 μm, between about 150 and 300 μm, between about 50 and 150 μm, between about 80 to 100 μm, between about 180 to 250 μm or between about 200 to 250 μm.

The methods and uses described herein are especially useful when performed in a suspension culture in a bioreactor. As described herein, the use of a permittivity probe allows monitoring inline the cell density without the need to obtain samples from the cell suspension or any other manual interaction with the cell suspension in a bioreactor. Accordingly, the measurement can be carried out in a bioreactor. In other words, the permittivity can be determined in a bioreactor. As used herein, the terms "reactor" and "bioreactor", which can be used interchangeably, refer to a closed culture vessel configured to provide a dynamic fluid environment for cell cultivation. The bioreactor may be stirred and/or agitated. Examples of agitated reactors include, but are not limited to, stirred tank bioreactors, wave-mixed/rocking bioreactors, up and down agitation bioreactors (i.e., agitation reactor comprising piston action), spinner flasks, shaker flasks, shaken bioreactors, paddle mixers, vertical wheel bioreactors. An agitated reactor may be configured to house a cell culture volume of between about 2 mL - 20,000 L. Preferred bioreactors may have a volume of up to 50 L. An exemplary bioreactor suitable for the method of the present invention is the ambr15 bioreactor available from Sartorius Stedim Biotech. The bioreactor can be a stainless steel or a single use bioreactor. The bioreactor can consist of a single vessel or can comprise several bioreactors in parallel. The single use bioreactor can be manufactured from glass or plastic. The single use bioreactor can be a stirred tank bioreactor or a rocking motion bioreactor. Examples: Sartorius STR, RM, ambr15, ambr 250. The pH of the culture medium may be controlled by the bioreactor, preferably controlled by $CO_2$ supply, and may be held in a range of 6.6 to 7.6, preferably at about 7.4.

The bioreactor may be a stirred bioreactor (STR). STRs are, e.g., available from Sartorius Stedim Biotech and include, but are not limited to, BIOSTAT® A/B/B-DCU/Cplus/D-DCU, ambr® 15 and ambr® 250. The bioreactor may be a rocking motion bioreactor (RM). RMs are, e.g., available from Sartorius Stedim Biotech and include, but are not limited to, BIOSTAT® RM and BIOSTAT® RM TX. The bioreactor may be a multi parallel bioreactor that is, e.g., available from Sartorius Stedim Biotech and include, but are not limited to, ambr® 15 and ambr® 250.

In some embodiments, the volume of the culture vessel in the bioreactor is from about 50 mL to about 20,000 L. In some embodiments, the volume of the culture vessel in the bioreactor is from about 50 mL to about 2,000 L. In some embodiments, the volume of the culture vessel in the bioreactor is from about 50 mL to about 200 L. In some embodiments, the volume of the culture vessel in the bioreactor is from about 50 mL to about 100 L. In some embodiments, the volume of the culture vessel in the bioreactor is from about 50 mL to about 50 L. In some embodiments, the volume of the culture vessel in the bioreactor is from about 50 mL to about 20 L. In some embodiments, the volume of the culture vessel in the bioreactor is from about 50 mL to about 10 L. In some embodiments, the volume of the culture vessel in the bioreactor is from about 50 mL to about 1 L. In some embodiments, the volume of the culture vessel in the bioreactor is from about 100 mL to about 10 L. In some embodiments, the volume of the culture vessel in the bioreactor is from about 100 mL to about 5

L. In some embodiments, the volume of the culture vessel in the bioreactor is from about 150 mL to about 1 L. In some embodiments, the volume of the culture vessel in the bioreactor is from about 1 L to about 1,000 L.

The cells may be any cells that can be cultivated in suspension. The cells can be selected from the group consisting of, primary cells, cells obtained from a tissue or an organ, immortalized cells, stem cells such as pluripotent stem cells or cells derived from stem cells, preferably cell derived from PSCs, preferably cell derived from iPSCs. The cells may be pluripotent stem cells. The cells may also be pluripotent stem cells selected from the group consisting of induced pluripotent stem cells (iPSC), embryonic stem cells (ESC), parthenogenetic stem cells (pPSC) and nuclear transfer derived PSCs (ntPSC), preferably iPSCs. Preferably, the cells are pluripotent stem cells, more preferably induced pluripotent stem cells (iPSCs), or cells derived from stem cells such as (i)PSCs. Examples of stem cells include, but are not limited to, pluripotent stem cells, cord blood stem cells, mesenchymal stem cell and/or hematopoietic stem cells, preferably pluripotent stem cells. Particularly preferred are induced pluripotent stem cells (iPSCs). "Cells derived from stem cells" relate to differentiated cells or cells differentiated into a specific cell type that are no longer capable of differentiating in any cell type of the body. Said cells derived from stem cells relate to cells, which are derived from the (pluripotent) stem cells used in the methods and uses of the invention and thus preferably do not include naturally occurring differentiated cells. Methods for the differentiation into different cell types starting from stem cells such as (i)PSCs are known to a person skilled in the art. "Cells derived from stem cells" may relate to heart cells and/or tissue, liver cells and/or tissue, kidney cells and/or tissue, brain cells and/or tissue, pancreatic cells and/or tissue, lung cells and/or tissue, skeletal muscle cells and/or tissue, gastrointestinal cells and/or tissue, neuronal cells and/or tissue, skin cells and/or tissue, bone cells and/or tissue, bone marrow, fat cells and/or tissue, connective cells and/or tissue, retinal cells and/or tissue, blood vessel cells and/or tissue, stromal cells or cardiomyocytes. Methods for generating heart tissue are known from WO 2015/025030 and WO 2015/040142. The cells may also be differentiated in the bioreactor or also outside of the bioreactor, e.g. to cardiomyocytes or stromal cells. These differentiated cells may also be cultured in a bioreactor making use of the method of the invention. Cells obtained from a tissue or an organ may be obtained from heart cells and/or tissue, liver cells and/or tissue, kidney cells and/or tissue, brain cells and/or tissue, pancreatic cells and/or tissue, lung cells and/or tissue, skeletal muscle cells and/or tissue, gastrointestinal cells and/or tissue, neuronal cells and/or tissue, skin cells and/or tissue, bone cells and/or tissue, bone marrow, fat cells and/or tissue, connective cells and/or tissue, retinal cells and/or tissue, blood vessel cells and/or tissue, stromal cells or cardiomyocytes.

The cells may be cells of a mammal, such as a human, a dog, a mouse, a rat, a pig, a non-human primate such as Rhesus macaque, baboon, cynomolgus macaque or common marmoset to name only a few illustrative examples. Preferably, the cells are human.

In multicellular organisms, "stem cells" are undifferentiated or partially differentiated cells that can differentiate into various types of cells and proliferate indefinitely to produce more of the same stem cell. They are usually distinguished from progenitor cells, which cannot divide indefinitely, and precursor or blast cells, which are usually committed to differentiating into one cell type. The term stem cells thus encompasses pluripotent stem cells but also multipotent (can differentiate into a number of cell types, but only those of a closely related family of cells), oligopotent stem cells (can differentiate into only a few cell types, such as lymphoid or myeloid stem cells) or unipotent stem cells such as satellite cells. Examples of stem cells include, but are not limited to, pluripotent stem cells, cord blood stem cells, mesenchymal stem cell and/or hematopoietic stem cells, preferably pluripotent stem cells. The term "pluripotent stem cell" (PSC) as used herein refers to cells that are able to differentiate into every cell type of the body. As such, pluripotent stem cells offer the unique opportunity to be differentiated into essentially any tissue or organ. Currently, the most utilized pluripotent cells are embryonic stem cells (ESC) or induced pluripotent stem cells (iPSC). Human ESC-lines were first established by Thomson and coworkers (Thomson et al. (1998), Science 282:1145-1147). Human ESC research recently enabled the development of a new technology to reprogram cells of the body into an ES-like cell. This technology was pioneered by Yamanaka and coworkers in 2006 (Takahashi & Yamanaka (2006), Cell, 126:663-676 and Takahashi et al. (2007), Cell, 131(5):861-72). Resulting induced pluripotent cells (iPSC) show a very similar behavior as ESC and, importantly, are also able to differentiate into every cell of the body. Thus, in one embodiment, the term iPSCs comprises ESC. In the context of the present invention, these pluripotent stem cells are however preferably not produced using a process which involves modifying the germ line genetic identity of human beings or which involves use of a human embryo for industrial or commercial purposes. Preferably, the pluripotent stem cells are of primate origin, more preferably human.

Suitable induced PSCs, can for example, be obtained from the NIH human embryonic stem cell registry, the European Bank of Induced Pluripotent Stem Cells (EBiSC), the Stem Cell Repository of the German Center for Cardiovascular Research (DZHK), or ATCC, to name only a few sources. Induced pluripotent stem cells are also available for commercial use, for example, from the NINDS Human Sequence and Cell Repository (https://stemcells.nindsgenetics.org) which is operated by the U.S. National Institute of Neurological Disorders and Stroke (NINDS) and distributes human cell resources broadly to academic and industry researchers. One illustrative example of a suitable cell line that can be used in the present invention is the cell line TC-1133, an induced (unedited) pluripotent stem cell that has been derived from a cord blood stem cell. This cell line is, e.g. directly available from NINDS, USA. Preferably, TC-1133 is GMP-compliant. Further exemplary iPSC cell lines that can be used in the present invention, include but are not limited to, the

Human Episomal iPSC Line of Gibco™ (order number A18945, Thermo Fisher Scientific), or the iPSC cell lines ATCC ACS-1004, ATCC ACS-1021, ATCC ACS-1025, ATCC ACS-1027 or ATCC ACS-1030 available from ATTC. Alternatively, any person skilled in the art of reprogramming can easily generate suitable iPSC lines by known protocols such as the one described by Okita et al, "A more efficient method to generate integration-free human iPS cells" Nature Methods, Vol.8 No.5, May 2011, pages 409-411 or by Lu et al "A defined xeno-free and feeder-free culture system for the derivation, expansion and direct differentiation of transgene-free patient-specific induced pluripotent stem cells", Biomaterials 35 (2014) 2816e2826.

The cells may be selected from the group consisting of TC-1133, the Human Episomal iPSC Line of Gibco ATCC ACS-1004, ATCC ACS-1021, ATCC ACS-1025, ATCC ACS-1027, and ATCC ACS-1030. Additionally or alternatively, the cells may be selected from the group consisting of HEK293, HEK293T, BHK 21, CHO, NS0, Sp2/0-Ag14.

As explained herein, the (induced) pluripotent stem cell that is used in the present invention can be derived from any suitable cell type (for example, from a stem cell such as a mesenchymal stem cell, or an epithelial stem cell or a differentiated cells such as fibroblasts) and from any suitable source (bodily fluid or tissue). Examples of such sources (body fluids or tissue) include cord blood, skin, gingiva, urine, blood, bone marrow, any compartment of the umbilical cord (for example, the amniotic membrane of umbilical cord or Wharton's jelly), the cord-placenta junction, placenta or adipose tissue, to name only a few. In one illustrative example, is the isolation of CD34-positive cells from umbilical cord blood for example by magnetic cell sorting using antibodies specifically directed against CD34 followed by reprogramming as described in Chou et al. (2011), Cell Research, 21:518-529. Baghbaderani et al. (2015), Stem Cell Reports, 5(4):647-659 show that the process of iPSC generation can be in compliance with the regulations of good manufacturing practice to generate cell line ND50039. Accordingly, the pluripotent stem cells preferably fulfil the requirements of the good manufacturing practice.

The present invention further relates to a method of expanding cells in cell aggregates in suspension culture, the method comprising: (i) Measuring the permittivity of the cell suspension; and (ii) Comparing the measured permittivity with a predetermined value that is indicative of the cell density, thereby determining the cell density. "Expanding" or "expansion of" cells as described herein describes an increase of cell number due to cell division.

The present invention further relates to the use of a permittivity probe for determining the cell density of a suspension cell culture comprising cell aggregates. The present invention further relates to the use of a permittivity probe in a method of the invention.

It is noted that as used herein, the singular forms "a", "an", and "the", include plural references unless the context clearly indicates otherwise. Thus, for example, reference to "a reagent" includes one or more of such different reagents and reference to "the method" includes reference to equivalent steps and methods known to those of ordinary skill in the art that could be modified or substituted for the methods described herein.

Unless otherwise indicated, the term "at least" preceding a series of elements is to be understood to refer to every element in the series. Those skilled in the art will recognize, or be able to ascertain using no more than routine experimentation, many equivalents to the specific embodiments of the invention described herein. Such equivalents are intended to be encompassed by the present invention.

The term "and/or" wherever used herein includes the meaning of "and", "or" and "all or any other combination of the elements connected by said term".

The term "less than" or in turn "more than" does not include the concrete number.

For example, less than 20 means less than the number indicated. Similarly, more than or greater than means more than or greater than the indicated number, e.g. more than 80 % means more than or greater than the indicated number of 80 %.

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integer or step. When used herein the term "comprising" can be substituted with the term "containing" or "including" or sometimes when used herein with the term "having". When used herein "consisting of" excludes any element, step, or ingredient not specified.

The term "including" means "including but not limited to". "Including" and "including but not limited to" are used interchangeably.

As used herein the terms "about", "approximately" or "essentially" mean within 20%, preferably within 15%, preferably within 10%, and more preferably within 5% of a given value or range. It also includes the concrete number, i.e. "about 20" includes the number of 20.

It should be understood that this invention is not limited to the particular methodology, protocols, material, reagents, and substances, etc., described herein and as such can vary. The terminology used herein is for the purpose of describing particular embodiments only, and is not intended to limit the scope of the present invention, which is defined solely by the claims.

## EXAMPLES

An even better understanding of the present invention and of its advantages will be evident from the following examples, offered for illustrative purposes only. The examples are not intended to limit the scope of the present invention in any way.

### Example 1: Permittivity measurements can be used for inline monitoring of cell density

To measure the permittivity, following material and equipment (see Table 1) is used according to the manufacturer's instructions:

**Table 1: Materials used in Example 1**

| Material and equipment | Detail |
|---|---|
| iPSCs | TC1133: TC1133 is a human iPS cell line that was generated by Lonza under cGMP-compliant conditions (Baghbaderani et al., 2015, 2016). |
| Bioreactor | UniVessel 0.5L (Sartorius) |
| Bioreactor controller | Biostat B – DCU II (Sartorius) |
| Permittivity measurement probe | BioPAT ViaMass (Sartorius) |
| Permittivity measurement probe controller | Standard Remote Futura (Futura) |
| Cell counter | Nucleocounter 200 (Chemometec) |

The cell-only aggregate suspension culture is performed as described in the following: TC1133 cells were seeded at 2.5 x $10^5$ cells/ml. Medium change was started at day 2 by adding 62% of current volume/day fresh medium. Cells were cultured at 37°C, pH 7.4, DO 23.8%. After every passage, the cells were seeded at 2.5 x $10^5$ cells/ml again.

The ViaMass probe is run in "Cell Culture" measurement mode at 580 kHz with BM220 PoIC, filter 30 and capacitance zero value 0 pF/cm. Before inoculation, the Viamass measurement is set to zero and the measurement is recorded throughout the entire culture. The cell concentration is assessed offline with a sample that was taken from the UniVessel bioreactor. The cell number is measured using the Nucleocounter 200 with the "Viability and cell count A100 and B" protocol. From day 1 onwards the iPSC aggregates need to be dissociated before measurement with the Nucleocounter 200. For this purpose, 1 mL of the sample is centrifuged for 1 min at 100 x g and the supernatant is removed. Subsequently, 1 ml TrypLE Express (Life Technologies) is added and incubated for 15 min at room temperature. The aggregates are resuspended by pipetting every 5 min. When the aggregates are dissociated the cell number is measured in the same way as described above.

Figure 1 shows the ViaMass permittivity and Nucleocounter 200 recordings during two separate culture runs. The cell concentration, which was determined with the Nucleocounter 200, correlates with the capacity, which was measured with the ViaMass permittivity probe. Importantly, the ViaMass permittivity recordings show dynamic changes such as plateaus and increases after changes in culture parameters. In Exemplary run No. 2, the permittivity measurement responded to an increase of the perfusion rate by a fast, subsequent rise in capacitance (Figure 1 B). These findings highlight the potential of permittivity measuring probes in cell-only aggregate suspension culture of iPSCs.

Importantly, the permittivity measurements did not correlate with the iPSC aggregate size (Figure 2). In exemplary run no. 1, the aggregate size was not affected by medium addition and the resulting changes in culture volume whereas the capacitance dynamically dropped (Figure 2A). Furthermore, in exemplary run no. 2 the cell and aggregate concentration was low after passaging on day 4 but the aggregate size increased as expected. This was not represented by the permittivity measurement (Figure 2B). The initial apparent correlation between the capacitance and the aggregate is a result of the constant culture volume and of the correlation between aggregate size and cell number of the aggregates.

Thus, permittivity measurements allow inline monitoring cell density of PSC aggregates. Importantly, the permittivity is surprisingly not influenced by increase of aggregate size, which increases the number of cell interactions. In sum, the inventors surprisingly found that permittivity measurements of PSC aggregates allow inline monitoring of cell density, which in turn allows reacting to cell density developments by process control.

## Example 2: Determination of a predetermined value based on a standard curve

In this example, the Inventors followed cell density of iPSC aggregate culture online by cell permittivity measurements (Viamass probe) and offline by standard cell counting (Nucleocounter). The following culture conditions were used:

**Experimental design and experiment progression:**

- Cells: TC1133 TL004, p4
- Seeding conditions: 450 ml with 2,5 x $10^5$ cells/ml.
- Medium change: Start at d2, perfusion 60%.
- Culture conditions: 37°C, pH 7.4, DO 23.8%, 45° blade angle, 120 rpm downstirr (day 0-1) and 100 rpm downstirr (d1-4).

Passage 1-3

- Seeding conditions: 320 ml with 2,5 x $10^5$ cells/ml.
- Medium change: Start at d2, perfusion 60% targeted.
- Culture conditions: 37 °C, pH 7.4, DO 23.8%, 45° blade angle, 120 rpm downstirr (day 0-1) and 100rpm downstirr (d1-end of passage).

**Materials**

Reagents and materials:

- StemMACS iPS-Brew XF, Basal Medium, Order no.: 130-107-086
- StemMACS iPS-Brew XF 50x Supplement; Order no.: 130-107-087

Devices

- Biostat B - DCU II: Type: BB-8841212
- Tower 3: Type: BB-8840152
    - pH sensor: Hamilton; Easyferm Plus VP 120
    - Oxygen Sensor: Hamilton; Oxyferm FDA VP 120
    - UniVessel 0.5 L
- pH meter: Multi 3510 IDS; Xylem Analytics Germany GmbH
- pH electrode: SenTix Micro 900P; WTW
- Nucleocounter NC-200 Type 900-0201
- Cellavista

Here, the Inventors compared an "internal" predetermined value (calculated by linear regression of cell capacitance and cell density data pairs of the same culture run) with "external" predetermined values, for which the predetermined value was derived from a reference suspension culture.

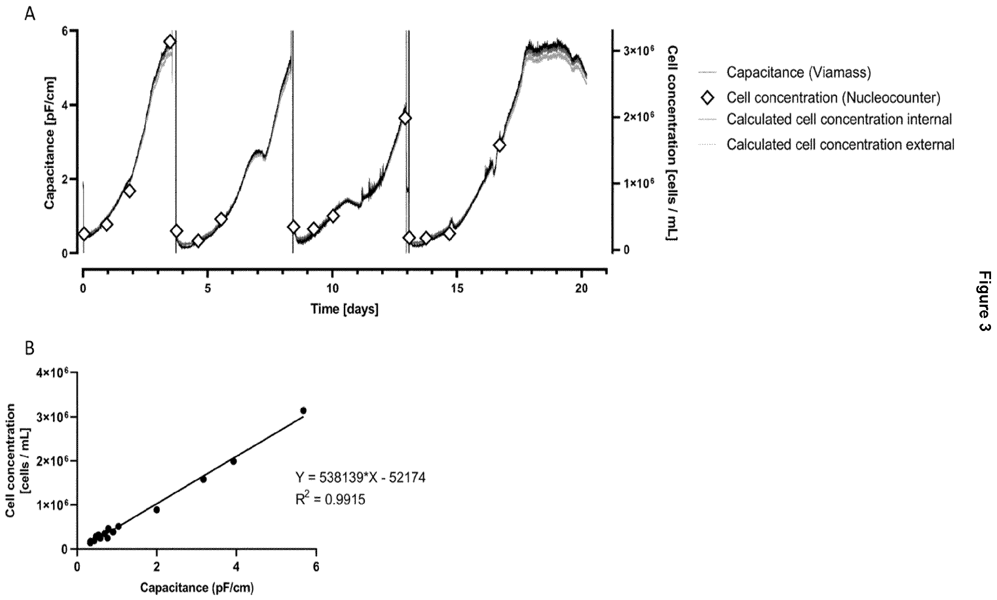

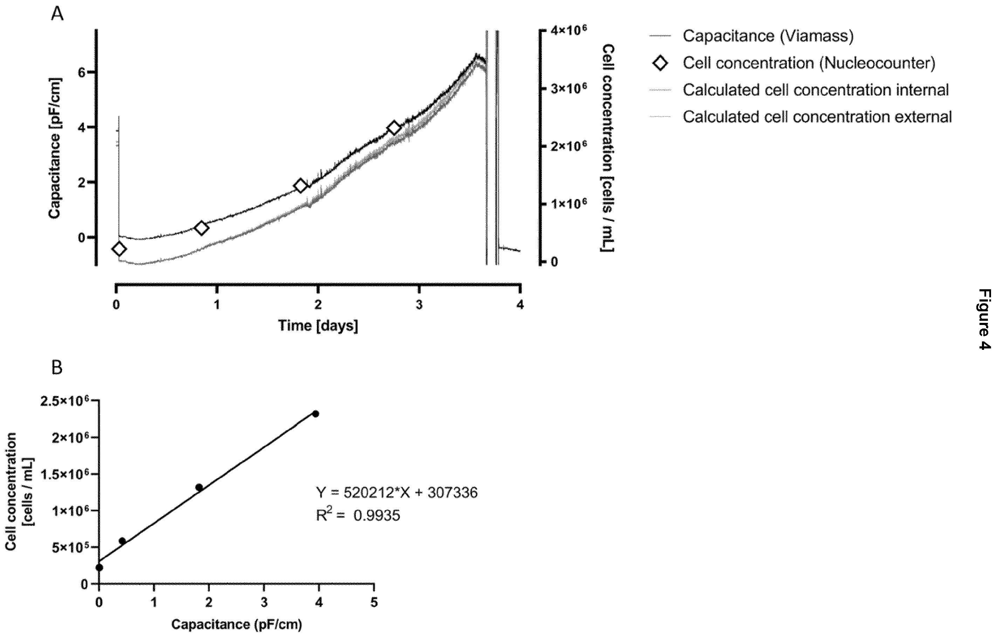

Fig. 3 shows one exemplary iPSC culture run, which was monitored inline and offline. As apparent from Fig. 3A, the capacitance and cell counting values correlate well during the complete run. The same is true for both the internal (see standard curve of Fig. 3B) and external (see standard curve of Fig. 4B) calculated cell concentration values, i.e. those values calculated based on the predetermined values. Fig. 3B shows a standard curve and a linear correlation of the permittivity measurements and the cell density obtained by counting the cells. The linear correlation yields a high $R^2$ value

showing that there is a linear correlation between cell density and permittivity.

Similarly, Fig. 4 shows a further exemplary iPSC culture run. Here, the same culture conditions were used. However, the cells were not passaged. Again, the cell permittivity, cell density and internal (see standard curve of Fig. 4B) and external (see standard curve of Fig. 3B) calculations correlate well (see Fig. 4A). Fig. 3B shows a standard curve and a linear correlation of the permittivity measurements and the cell density obtained by counting the cells. The linear correlation yields a high $R^2$ value showing that there is a linear correlation between cell density and permittivity.

Importantly, it is not necessary to calculate the predetermined value for each and every cell culture run. In contrast, the predetermined value is comparable between similar culture conditions as shown above. Thus, it is possible to use a predetermined value obtained from a reference suspension culture. This allows an easy online measurement of cell density of PSC aggregates while avoiding taking samples for manual or automated cell counting. The method of the invention thus provides an additional important value of the cell culture. Most importantly, the cell density rate is provided in real-time and thus can be taken into account for process control.

**REFERENCES**

Baghbaderani, B.A., Tian, X., Neo, B.H., Burkall, A., Dimezzo, T., Sierra, G., Zeng, X., Warren, K., Kovarcik, D.P., Fellner, T., et al. (2015). cGMP-Manufactured Human Induced Pluripotent Stem Cells Are Available for Pre-clinical and Clinical Applications. Stem Cell Reports 5, 647-659.

Baghbaderani, B.A., Syama, A., Sivapatham, R., Pei, Y., Mukherjee, O., Fellner, T., Zeng, X., and Rao, M.S. (2016). Detailed Characterization of Human Induced Pluripotent Stem Cells Manufactured for Therapeutic Applications. Stem Cell Rev and Rep 12, 394-420.

Chen, V.C., Couture, S.M., Ye, J., Lin, Z., Hua, G., Huang, H.-I.P., Wu, J., Hsu, D., Carpenter, M.K., and Couture, L.A. (2012). Scalable GMP compliant suspension culture system for human ES cells. Stem Cell Research 8, 388-402.

Halloin, C., Coffee, M., Manstein, F., and Zweigerdt, R. (2019). Production of Cardiomyocytes from Human Pluripotent Stem Cells by Bioreactor Technologies. In Cell-Based Assays Using IPSCs for Drug Development and Testing, C.-F. Mandenius, and J.A. Ross, eds. (New York, NY: Springer New York), pp. 55-70.

Hemmi, N., Tohyama, S., Nakajima, K., Kanazawa, H., Suzuki, T., Hattori, F., Seki, T., Kishino, Y., Hirano, A., Okada, M., et al. (2014). A Massive Suspension Culture System With Metabolic Purification for Human Pluripotent Stem Cell-Derived Cardiomyocytes. STEM CELLS Translational Medicine 3, 1473-1483.

Jiang, Y., Langenberg, K., Borgdorff, V., Duńska, M., Post, R., Bartulos, O., Doornbos, M., Braam, S., Reijerkerk, A., and Rasche, U. (2019). Controlled, Large-Scale Manufacturing of hiPSC-Derived Cardiomyocytes in Stirred-Tank Bioreactors. 12.

Justice, C., Brix, A., Freimark, D., Kraumec, M., Pfromm, P., Eichenmueller, B., Czermak, P. (2011). Process control in cell culture technology using dielectric spectroscopy. Biotechnology Advances 29(4), 391-401.

Kempf, H., Kropp, C., Olmer, R., Martin, U., and Zweigerdt, R. (2015). Cardiac differentiation of human pluripotent stem cells in scalable suspension culture. Nature Protocols 10, 1345-1361.

Kropp, C., Kempf, H., Halloin, C., Robles-Diaz, D., Franke, A., Scheper, T., Kinast, K., Knorpp, T., Joos, T.O., Haverich, A., et al. (2016). Impact of Feeding Strategies on the Scalable Expansion of Human Pluripotent Stem Cells in Single-Use Stirred Tank Bioreactors. STEM CELLS Translational Medicine 5, 1289-1301.

Kropp, C., Massai, D., and Zweigerdt, R. (2017). Progress and challenges in large-scale expansion of human pluripotent stem cells. Process Biochemistry 59, 244-254.

**Claims**

**1.** A method of measuring cell density in a cell suspension comprising cell aggregates, the method comprising

(i) Measuring the permittivity of the cell suspension;
(ii) Comparing the measured permittivity with a predetermined value that is indicative of the cell density, thereby determining the cell density.

**2.** The method of claim 1, wherein the measuring is carried out in a bioreactor.

**3.** The method of claim 2, wherein the bioreactor is a stirred bioreactor, a rocking motion bioreactor and/or a multi parallel bioreactor.

**4.** The method of claim 2 or 3, wherein the cell density of the suspension culture is measured inline and in real time.

**5.** The method of any one of the preceding claims, wherein the measurement of the permittivity is carried out using a permittivity probe; and/or wherein the permittivity measurement is carried out by using dielectric spectroscopy.

**6.** The method of any one of the preceding claims, wherein the cells are selected from the group consisting of, primary cells, cells obtained from a tissue or an organ, immortalized cells, stem cells such as pluripotent stem cells, or cells derived from stem cells, wherein the cells preferably are pluripotent stem cells, wherein the cells more preferably are pluripotent stem cells selected from the group consisting of induced pluripotent stem cells (iPSC), embryonic stem cells (ESC), parthenogenetic stem cells (pPSC) and nuclear transfer derived PSCs (ntPSC), preferably iPSCs.

**7.** The method of any one of the preceding claims, wherein the conversion factor is obtained by

(a) Measuring the permittivity and the cell density at at least two, preferably at least three, different cell densities of a reference suspension culture;
(b) Correlating the measured cell permittivity of the reference suspension culture with the cell density, thereby determining the predetermined value that is indicative of the cell density.

**8.** The method of claim 7, wherein the correlation provides a linear correlation.

**9.** The method of claim 7 or 8, wherein the reference suspension culture and the suspension culture are

(i) from a similar or the same cell type, cell line, tissue or organ;
(ii) cultured using the same culture medium; and/or
(iii) cultured in a similar or the same bioreactor.

**10.** The method of any one of the preceding claims, wherein the cell density is viable cell density.

**11.** Use of a permittivity probe in a method as defined in any of claims 1-10.

**12.** The method of any one of claims 1-10 or the use of claim 11 wherein the cells are not in microcarrier culture.

**Patentansprüche**

**1.** Verfahren zur Messung der Zelldichte in einer Zellsuspension, welche Zellaggregate umfasst, wobei das Verfahren umfasst:

(i) Messen der Permittivität der Zellsuspension;
(ii) Vergleichen der gemessenen Permittivität mit einem vorherbestimmten Wert, welcher über die Zelldichte Aufschluss gibt, wodurch die Zelldichte bestimmt wird.

**2.** Verfahren nach Anspruch 1, wobei das Messen in einem Bioreaktor durchgeführt wird.

**3.** Verfahren nach Anspruch 2, wobei der Bioreaktor ein Rührkesselreaktor, ein Rocking-Motion-Bioreaktor und/oder ein multiparalleler Bioreaktor ist.

**4.** Verfahren nach Anspruch 2 oder 3, wobei die Zelldichte der Suspensionskultur inline und in Echtzeit gemessen wird.

5. Verfahren nach einem der vorangegangenen Ansprüche, wobei die Messung der Permittivität mittels einer Permittivitätssonde durchgeführt wird; und/oder wobei die Permittivitätsmessung mittels dielektrischer Spektroskopie durchgeführt wird.

6. Verfahren nach einem der vorangegangenen Ansprüche, wobei die Zellen ausgewählt sind aus der Gruppe bestehend aus Primärzellen, Zellen aus einem Gewebe oder einem Organ, immortalisierten Zellen, Stammzellen wie pluripotenten Stammzellen oder Zellen, welche von Stammzellen abgeleitet sind, wobei die Zellen vorzugsweise pluripotente Stammzellen sind, wobei die Zellen vorzugsweise pluripotente Stammzellen sind, welche aus der Gruppe ausgewählt sind, die aus induzierten pluripotenten Stammzellen (iPSC), embryonalen Stammzellen (ESC), parthenogenetischen Stammzellen (pPSC) und nukleartransfer-abgeleiteten PSCs (ntPSC), vorzugsweise iPSCs, besteht.

7. Verfahren nach einem der vorangegangenen Ansprüche, wobei der Umrechnungsfaktor erhalten wird durch

   (a) Messen der Permittivität und der Zelldichte bei mindestens zwei, vorzugsweise mindestens drei, verschiedenen Zelldichten einer Referenz-Suspensionskultur;
   (b) Korrelieren der gemessenen Zellpermittivität der Referenz-Suspensionskultur mit der Zelldichte, wodurch der vorherbestimmte Wert bestimmt wird, der über die Zelldichte Aufschluss gibt.

8. Verfahren nach Anspruch 7, wobei die Korrelation eine lineare Korrelation bereitstellt.

9. Verfahren nach Anspruch 7 oder 8, wobei die Referenz-Suspensionskultur und die Suspensionskultur

   (i) aus einem ähnlichen oder demselben Zelltyp, einer ähnlichen oder derselben Zelllinie, einem ähnlichen oder demselben Gewebe oder Organ sind;
   (ii) unter Verwendung desselben Kulturmediums kultiviert werden; und/oder
   (iii) in einem ähnlichen oder demselben Bioreaktor kultiviert werden.

10. Verfahren nach einem der vorangegangenen Ansprüche, wobei die Zelldichte eine Lebendzelldichte ist.

11. Verwendung einer Permittivitätssonde in einem Verfahren nach einem der Ansprüche 1-10.

12. Verfahren nach einem der Ansprüche 1-10, oder Verwendung nach Anspruch 11, wobei die Zellen nicht in einer Mikroträgerkultur sind.

**Revendications**

1. Procédé de mesure de la densité cellulaire dans une suspension cellulaire comprenant des agrégats cellulaires, le procédé comprenant

   (i) Mesurer la permittivité de la suspension cellulaire;
   (ii) Comparer la permittivité mesurée avec une valeur prédéterminée qui est indicative de la densité cellulaire, déterminant ainsi la densité cellulaire.

2. Le procédé selon la revendication 1, dans lequel la mesure est effectuée dans un bioréacteur.

3. Le procédé selon la revendication 2, dans lequel le bioréacteur est un bioréacteur agité, un bioréacteur à mouvement de basculement et/ou un bioréacteur multiparallèle.

4. Le procédé selon la revendication 2 ou 3, dans lequel la densité cellulaire de la culture en suspension est mesurée en ligne et en temps réel.

5. Le procédé selon l'une des revendications précédentes, dans lequel la mesure de la permittivité est effectuée en utilisant une sonde de permittivité ;et/ou où la mesure de la permittivité est effectuée en utilisant la spectroscopie diélectrique.

6. Le procédé selon l'une des revendications précédentes, dans lequel les cellules sont sélectionnées parmi le groupe

constitué de cellules primaires, de cellules obtenues à partir d'un tissu ou d'un organe, de cellules immortalisées, de cellules souches telles que les cellules souches pluripotentes, ou de cellules dérivées de cellules souches, où les cellules sont de préférence des cellules souches pluripotentes, où les cellules sont de préférence des cellules souches pluripotentes sélectionnées parmi le groupe constitué des cellules souches pluripotentes induites (iPSC), de cellules souches embryonnaires (ESC), de cellules souches parthénogénétiques (pPSC) et PSC dérivées du transfert nucléaire (ntPSC), de préférence des iPSC.

**7.** Le procédé selon l'une des revendications précédentes, dans lequel le facteur de conversion est obtenu par

(a) Mesurer la permittivité et la densité cellulaire à au moins deux, de préférence au moins trois, densités cellulaires différentes d'une culture de suspension de référence ;
(b) Mise en corrélation entre la permittivité cellulaire mesurée de la culture de suspension de référence et la densité cellulaire, déterminant ainsi la valeur prédéterminée qui est indicative de la densité cellulaire.

**8.** Le procédé selon la revendication 7, dans lequel la corrélation fournit une corrélation linéaire.

**9.** Le procédé selon la revendication 7 ou 8, dans lequel la culture de suspension de référence et la culture de suspension sont

(i) à partir d'un même ou similaire type cellulaire, d'une même ou similaire lignée cellulaire, d'un même ou similaire tissu ou organe;
(ii) cultivé en utilisant le même milieu de culture ; et/ou
(iii) cultivé dans le même bioréacteur ou dans un bioréacteur similaire.

**10.** Le procédé selon l'une des revendications précédentes, dans lequel la densité cellulaire est une densité cellulaire viable.

**11.** Utilisation d'une sonde de permittivité dans un procédé selon l'une quelconque des revendications 1 à 10.

**12.** Le procédé selon l'une des revendications 1 à 10 ou l'utilisation selon la revendication 11, où les cellules ne sont pas en culture de microporteurs.

**Figure 1**

A
BioPAT ViaMass
Nucleocounter 200
Capacitance [pF/cm]
-305
-306
-307
-308
-309
-310
Feed
(60 %)
Feed
(60 %)
*
Cell concentration [cells / mL]
3×10⁶
2×10⁶
1×10⁶
0
Time [days]
0
1
2
3
4
B
BioPAT ViaMass
Nucleocounter 200
Capacitance [pF/cm]
6
4
2
0
-2
*
Cell concentration [cells / mL]
2×10⁶
1×10⁶
0
-1×10⁶
Time [days]
0
5
10

Figure 2

A
B
BioPAT ViaMass
Cellavista
Feed
(60 %)
Feed
(60 %)
*
Capacitance [pF/cm]
Aggregate size [µm]
Time [days]
-305
-306
-307
-308
-309
-310
150
100
0
1
2
3
4
6
4
2
0
-2
2×10²
1.5×10²
1×10²
5
10

Figure 3

A
Capacitance [pF/cm]
6
4
2
0
Cell concentration [cells / mL]
3×10^6
2×10^6
1×10^6
0
Time [days]
0
5
10
15
20
Capacitance (Viamass)
Cell concentration (Nucleocounter)
Calculated cell concentration internal
Calculated cell concentration external
B
Cell concentration [cells / mL]
4×10^6
3×10^6
2×10^6
1×10^6
0
Capacitance (pF/cm)
0
2
4
6
Y = 538139*X - 52174
R^2 = 0.9915

Figure 4

A
Capacitance [pF/cm]
Time [days]
Cell concentration [cells / mL]
Capacitance (Viamass)
Cell concentration (Nucleocounter)
Calculated cell concentration internal
Calculated cell concentration external
B
Cell concentration [cells / mL]
Capacitance (pF/cm)
Y = 520212*X + 307336
R2 = 0.9935

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 21152718
- US 2015019140 A
- WO 2015025030 A
- WO 2015040142 A


### Non-patent literature cited in the description


- **THOMSON et al.** *Science*, 1998, vol. 282, 1145-1147
- **TAKAHASHI** ; **YAMANAKA**. *Cell*, 2006, vol. 126, 663-676
- **TAKAHASHI et al.** *Cell*, vol. 131 (5), 861-72
- **OKITA et al.** A more efficient method to generate integration-free human iPS cells. *Nature Methods*, May 2011, vol. 8 (5), 409-411
- **LU et al.** A defined xeno-free and feeder-free culture system for the derivation, expansion and direct differentiation of transgene-free patient-specific induced pluripotent stem cells. *Biomaterials*, 2014, vol. 35, 2816e2826
- **CHOU et al.** *Cell Research*, 2011, vol. 21, 518-529
- **BAGHBADERANI et al.** *Stem Cell Reports*, 2015, vol. 5 (4), 647-659
- **BAGHBADERANI, B.A.** ; **TIAN, X.** ; **NEO, B.H.** ; **BURKALL, A.** ; **DIMEZZO, T.** ; **SIERRA, G.** ; **ZENG, X.** ; **WARREN, K.** ; **KOVARCIK, D.P.** ; **FELLNER, T. et al.** cGMP-Manufactured Human Induced Pluripotent Stem Cells Are Available for Pre-clinical and Clinical Applications. *Stem Cell Reports*, 2015, vol. 5, 647-659
- **BAGHBADERANI, B.A.** ; **SYAMA, A.** ; **SIVAPATHAM, R.** ; **PEI, Y.** ; **MUKHERJEE, O.** ; **FELLNER, T.** ; **ZENG, X.** ; **RAO, M.S.** Detailed Characterization of Human Induced Pluripotent Stem Cells Manufactured for Therapeutic Applications. *Stem Cell Rev and Rep*, 2016, vol. 12, 394-420
- **CHEN, V.C.** ; **COUTURE, S.M.** ; **YE, J.** ; **LIN, Z.** ; **HUA, G.** ; **HUANG, H.-I.P.** ; **WU, J.** ; **HSU, D.** ; **CARPENTER, M.K.** ; **COUTURE, L.A.** Scalable GMP compliant suspension culture system for human ES cells. *Stem Cell Research*, 2012, vol. 8, 388-402
- Production of Cardiomyocytes from Human Pluripotent Stem Cells by Bioreactor Technologies. **HALLOIN, C.** ; **COFFEE, M.** ; **MANSTEIN, F.** ; **ZWEIGERDT, R.** Cell-Based Assays Using IPSCs for Drug Development and Testing. Springer, 2019, 55-70
- **HEMMI, N.** ; **TOHYAMA, S.** ; **NAKAJIMA, K.** ; **KANAZAWA, H.** ; **SUZUKI, T.** ; **HATTORI, F.** ; **SEKI, T.** ; **KISHINO, Y.** ; **HIRANO, A.** ; **OKADA, M. et al.** A Massive Suspension Culture System With Metabolic Purification for Human Pluripotent Stem Cell-Derived Cardiomyocytes. *STEM CELLS Translational Medicine*, 2014, vol. 3, 1473-1483
- **JIANG, Y.** ; **LANGENBERG, K.** ; **BORGDORFF, V.** ; **DUŃSKA, M.** ; **POST, R.** ; **BARTULOS, O.** ; **DOORNBOS, M.** ; **BRAAM, S.** ; **REIJERKERK, A.** ; **RASCHE, U.** *Controlled, Large-Scale Manufacturing of hiPSC-Derived Cardiomyocytes in Stirred-Tank Bioreactors*, 2019, 12
- **JUSTICE, C.** ; **BRIX, A.** ; **FREIMARK, D.** ; **KRAUMEC, M.** ; **PFROMM, P.** ; **EICHENMUELLER, B.** ; **CZERMAK, P.** Process control in cell culture technology using dielectric spectroscopy. *Biotechnology Advances*, 2011, vol. 29 (4), 391-401
- **KEMPF, H.** ; **KROPP, C.** ; **OLMER, R.** ; **MARTIN, U.** ; **ZWEIGERDT, R.** Cardiac differentiation of human pluripotent stem cells in scalable suspension culture. *Nature Protocols*, 2015, vol. 10, 1345-1361
- **KROPP, C.** ; **KEMPF, H.** ; **HALLOIN, C.** ; **ROBLES-DIAZ, D.** ; **FRANKE, A.** ; **SCHEPER, T.** ; **KINAST, K.** ; **KNORPP, T.** ; **JOOS, T.O.** ; **HAVERICH, A. et al.** Impact of Feeding Strategies on the Scalable Expansion of Human Pluripotent Stem Cells in Single-Use Stirred Tank Bioreactors. *STEM CELLS Translational Medicine*, 2016, vol. 5, 1289-1301
- **KROPP, C.** ; **MASSAI, D.** ; **ZWEIGERDT, R.** Progress and challenges in large-scale expansion of human pluripotent stem cells. *Process Biochemistry*, 2017, vol. 59, 244-254